# EUROPEAN PATENT SPECIFICATION

(11) **EP 4 181 813 B1**
(45) Date of publication and mention of the grant of the patent: **31.12.2025**
(21) Application number: 21762167.1
(22) Date of filing: 14.07.2021
(51) Int. Cl.: A61B 34/10, A61B 5/00, A61B 5/11, A61B 34/20, A61B 90/00, A61F 2/42, A61B 90/50, A61F 2/46

(54) **DYNAMIC JOINT ANALYSIS FOR JOINT REPLACEMENT**
DYNAMISCHE GELENKANALYSE FÜR GELENKERSATZ
ANALYSE DYNAMIQUE D'ARTICULATION POUR REMPLACEMENT D'ARTICULATION

(30) Priority: 14.07.2020 US 202063051660 P
(43) Date of publication of application: 24.05.2023
(73) Proprietor: Howmedica Osteonics Corp., Mahwah, NJ 07430 (US)
(72) Inventor: REYNOLDS, David, Memphis, Tennessee 38177 (US); MOORE, Jesse G., Memphis, Tennessee 38177 (US)
(74) Representative: Zimmermann & Partner Patentanwälte mbB
(86) International application number: PCT/US2021/041672
(87) International publication number: WO 2022/015877

(56) References cited:
- EP-A2- 3 646 809
- WO-A1-2013/020026
- WO-A1-2018/140429
- WO-A1-2019/148154
- US-A1- 2019 167 352
- US-A1- 2019 380 792
- ALBERTO LEARDINI ET AL: "Biomechanics of the natural, arthritic, and replaced human ankle joint", JOURNAL OF FOOT AND ANKLE RESEARCH, BIOMED CENTRAL LTD, LONDON UK, vol. 7, no. 8, 6 February 2014 (2014-02-06), pages 1 - 16, XP021176387, ISSN: 1757-1146, DOI: 10.1186/1757-1146-7-8
- BROCKETT CLAIRE L ET AL: "Biomechanics of the ankle", ORTHOPAEDICS AND TRAUMA, ELSEVIER, AMSTERDAM, NL, vol. 30, no. 3, 16 June 2016 (2016-06-16), pages 232 - 238, XP029668537, ISSN: 1877-1327, DOI: 10.1016/J.MPORTH.2016.04.015

## Description

This application claims priority to U.S. Provisional Patent Application No. 63/051,660, filed July 14, 2020 and entitled "DYNAMIC JOINT ANALYSIS FOR JOINT REPLACEMENT".

### BACKGROUND

Surgical joint repair procedures involve repair and/or replacement of a damaged or diseased joint. A surgical joint repair procedure, such as joint arthroplasty as an example, may involve replacing the damaged joint with a prosthetic that is implanted into the patient's bone. A surgeon may analyze damaged bone to assist with prosthetic selection, design and/or positioning, as well as surgical steps to prepare bone or tissue to receive or interact with a prosthetic.

WO 2019/148154 A1 describes systems, devices and methods for performing a surgical step or surgical procedure with visual guidance using an optical head mounted display. Systems, devices and methods for displaying, placing, fitting, sizing, selecting, aligning, moving a virtual implant on a physical anatomic structure of a patient and, optionally, modifying or changing the displaying, placing, fitting, sizing, selecting, aligning, moving, for example based on kinematic information, are described. US 2019/380792 A1 describes that an example method includes displaying, via a visualization device and overlaid on a portion of an anatomy of a patient viewable via the visualization device, a virtual model of the portion of the anatomy obtained from a virtual surgical plan for an orthopedic joint repair surgical procedure to attach a prosthetic to the anatomy; and displaying, via the visualization device and overlaid on the portion of the anatomy, a virtual guide that guides at least one of preparation of the anatomy for attachment of the prosthetic or attachment of the prosthetic to the anatomy. US 2019/167352 A1 describes a method of designing an orthopedic implant comprising: (a) iteratively evaluating possible shapes of a dynamic orthopedic implant using actual anatomical shape considerations and kinematic shape considerations; and, (b) selecting a dynamic orthopedic implant shape from one of the possible shapes, where the dynamic orthopedic implant shape selected satisfies predetermined kinematic and anatomical constraints.

### SUMMARY

The invention is defined by the appended claims.

Total ankle replacement surgery (sometimes referred to as an "ankle arthroplasty") involves removing cartilage bone from the ankle joint and replacing it with one or more implants. To understand the actual kinematic behavior of the knee, leg, and ankle of a patient, a computing system may be configured to perform dynamic ankle analysis of the ankle joint of the patient. Dynamic ankle analysis may include identifying one or more ankle joint attributes to understand the kinematic axis of rotation of the ankle to improve planning and execution of total ankle replacement surgery. Additionally, dynamic ankle analysis may pinpoint causes of physical limitations due to impinging bone structures (e.g., existing bone, osteophytes, etc.), soft tissue impingement and limitations caused by over-tension constraints, and degenerative conditions.

Dynamic ankle analysis may employ patient-specific data that is indicative of the pre-operative motion of the ankle of the patient to determine the kinematic properties (e.g., the kinematic flexion axis, the kinematic inversion/eversion axis, the kinematic abduction/adduction axis, the soft tissue constraints, etc.) of the ankle of the patient. This data may be obtained, for example, using a clinical gait lab with inverse dynamics and/or multiple images of ankle in different potions about the various kinematic axes. In some examples, a computing system is configured to utilize this patient specific data to morph a standardized, three-dimensional model of a foot and ankle into a patient-specific three-dimensional model.

Using any of these techniques, a computing system can be configured to identify a current ankle kinematic axis of the patient, generate a surgery plan that selects an implant and determine other surgical interventions, and/or provide interoperative guidance (e.g., via a mixed reality system, etc.) to transition from the current kinematic axis to a desired kinematic axis during a surgical procedure.

An example system includes an analysis device and a surgical planning system. The analysis device receives patient specific data indicative of pre-operative motion associated with an ankle of a patient. The analysis device determines, based on the patient specific data, a current kinematic axis of the motion associated with the ankle, and determines a target kinematic axis of motion for the ankle. The target kinematic axis being different than the current kinematic axis. Additionally, the analysis device generates a transform function between the current kinematic axis and the target kinematic axis. The surgical planning system generates, based on the transform function, a recommended surgical intervention that adjusts tissue associated with the ankle to achieve the target kinematic axis of motion.

An example computer readable medium comprises instructions that, when executed, cause one or more processing circuits to, in response to receiving patient specific data indicative of pre-operative motion associated with an ankle of a patient, determine, based on the patient specific data, a current kinematic axis of the motion associated with the ankle. The instructions, when executed also cause the one or more processing circuits to determine a target kinematic axis of motion for the ankle, the target kinematic axis being different than the current kinematic axis, and generate a transform function between the current kinematic axis and the target kinematic axis. Additionally, the instructions, when executed also cause the one or more processing circuits to generate, based on the transform function, a recommended surgical intervention that adjusts tissue associated with the ankle to achieve the target kinematic axis of motion.

The details of various examples of the disclosure are set forth in the accompanying drawings and the description below. Various features, objects, and advantages will be apparent from the description, drawings, and claims.

### BRIEF DESCRIPTION OF DRAWINGS

FIG. 1 is a block diagram illustrating an example computing device configured to implement the techniques of this disclosure.
FIGS. 2A, 2B, and 2C illustrate example positions in which to obtain images of an ankle about a kinematic flexion axis for dynamic ankle analysis in accordance with the teachings of this disclosure.
FIGS. 3A and 3B illustrate an example joint depicting an initial kinematic axis and a target kinematic axis.
FIGS. 4A, 4B, and 4C illustrates an example joint depicting visual representations of impingement to movement.
FIG. 5 is a flowchart of an example method to generate a surgical plan based on a dynamic joint analysis in accordance with the teachings of this disclosure.
FIG. 6 is a flowchart of an example method to intraoperatively change a surgical plan based on a dynamic joint analysis in accordance with the teachings of this disclosure.
FIG. 7 is a flowchart of an example method to generate a patient specific computational model to determine an initial kinematic axis of a joint in accordance with the teachings of this disclosure.

### DETAILED DESCRIPTION

The goal of ankle replacement surgery is to relieve pain (e.g., from arthritis or an ankle injury, etc.) and/or improving ankle range of motion to preserve angle function and so that the related joints do not experience additional stress. External bony references (such as the trans-malleolar axis, the foot orientation, the direction of the second toe, and/or the orientation of the gutter surfaces, etc.) can be used as surrogates for the ankle axis. However, these external bony references do not provide a complete understanding of ankle kinematic geometry and are, at best, approximations. For example, there can be a large variation in internal/external orientation of the kinematic flexion axis of the ankle relative to foot, knee, and other landmarks. Additionally, bony anatomy makes up some of the constraint of the ankle motion, but ligament, tendon, muscles, and loading conditions also affect the kinematics. Understanding the actual kinematic behavior of the knee, leg, and ankle of a patient can improve the outcomes of patients that receive total ankle replacement surgery.

Assessment methods may include use of a static reference (e.g., an image produced by X-ray imaging, CT scan imaging, MRI imaging, etc.) with limited ability to characterize mobility of a dynamic joint (e.g., an ankle, etc.). For example, a static scan may be useful in determining the appropriate alignment of an implant in that static position, such an image is unable to fully characterize the constraints of motion or identify the causes of limited dynamic motion (e.g., flexion, extension, and rotation). While a static scan may provide information to perform total ankle replacement surgery, as describe below, dynamic ankle analysis may facilitate analysis and planning that includes consideration of the kinematic properties of the ankle to obtain appropriate implant positioning though the desired range of motion of the joint.

As described herein, a computing system may be configured to perform dynamic ankle analysis of the ankle joint of the patient to identify ankle joint properties and understand the kinematic axis of rotation of the ankle. While, in general, the techniques described below are described in terms of dynamic ankle analysis, techniques described herein may be used to characterize other joints of the body. Dynamic ankle analysis may provide analysis of current physical limitations of the ankle joint of a patient due to factors such as bone geometry, impinging bone structures (e.g., existing bone, osteophytes, etc.), soft tissue impingement and limitations caused by over-tension constraints, and/or degenerative conditions (such as osteoarthritis, etc.). A computer system may perform dynamic ankle analysis using patient-specific data that is indicative of the pre-operative motion of the ankle of the patient to determine the kinematic properties (e.g., the kinematic flexion axis, the kinematic inversion/eversion axis, the kinematic abduction/adduction axis, the soft tissue constraints, etc.) of the ankle of the patient. In some examples, the computer system may perform dynamic ankle analysis using a clinical gait lab with inverse dynamics.

Additionally or alternatively, in some examples, a computer system may perform dynamic ankle analysis based on multiple images of ankle in different potions about the kinematic axis of interest. For example, dynamic ankle analysis may use an CT scan of the ankle in a dorsiflexion position, a neutral position, and a plantarflexion position to determine location and motion about the kinematic flexion axis (sometime referred to as the "coronal axis" or the "ML axis"). In some examples, the images may be taken while the ankle is loaded (e.g., under a weight normally applied to the ankle while the joint is in use). Using the multiple images of the ankle about the axis of interest, the computer system may analyze the relative transform between each of the bones near the ankle to determine the translational and rotational flexibility of the joint. For example, the computer system may calculate a relative transformation of the tibia and talus from each of the CT images to compute the motion of the talus relative to the tibia to determine the kinematic flexion axis. This information is used to determine a desired kinematic state. This analysis may, for example, determine if the joint is hypo-mobile or hyper-mobile compared to the mobility of a statistically normal joint (e.g., taking into account height, weight, gait, etc. of the patient). For example, when the dynamic ankle analysis determines that the current kinematic axis is access is hypo-mobile or hyper-mobile, a desired kinematic axis of motion may be to return to a normal joint mobility. As used herein, normal joint mobility or normal kinematic axis refers to a statistical average joint mobility or kinematic axis of patients with similar demographic and physical characteristics.

In some examples, patient specific data obtain through the clinical gait lab and/or the multiple image analysis is used to morph a standardized, three-dimensional computational model of a foot and an ankle into a patient-specific three-dimensional computational model. The standardized computational model includes an established kinematic gait cycle. Additionally, the standardized computational model includes definitions of ligaments and tendons and corresponding attachment points. For example, the images of the joint may be turned into a three-dimensional (3D) model and the 3D model is used to morph the standardized model into a patient-specific model. The patient-specific computational model may then be run to compute the initial kinematic axes of the ankle. The patient-specific computational model may also highlight range of motion constraints. For example, the patient-specific computational model may highlight insufficient plantarflexion or excessive coronal tilt during walking. The patient-specific computation model may highlight impingements that prevent the joint moving on a normal kinematic axis, such as bony impingements caused by ankle gutters or tendon that are too tight.

These techniques may enable a computer system to identify a current ankle kinematic axis of the patient to generate a surgery plan that selects an implant and determine other surgical interventions and/or provide interoperative guidance (e.g., via a mixed reality system, etc.) to transition from the current kinematic axis to a desired (target) kinematic axis. For example, the computational model may run patient-specific simulations with different implants and/or changing different impingements to generate a transform function to achieve the desired kinematic axes starting from the current kinematic axes. This transform function is translated into one or more recommended surgical interventions to form the basis of a surgical plan. In some examples, a mixed reality system may present the surgical plan to the surgeon before, during, and/or after the joint replacement procedure. Additionally or alternatively, the mixed reality system may the highlight range of motion constraints and/or impingements that prevent the joint moving on a normal kinematic axis to facilitate the surgeon making interoperative decisions while enacting the surgical plan.

In some examples, interoperative tracking identifies where limitations of motion are occurring. In some examples, images captured during a surgery are analyzed to change the surgical plan or to provide more detailed guidance. For example, images capture during surgery may detect a bony impingement at a certain flexion angle. The mixed reality system may highlight the intraoperatively detected bony impingement. As another example, the images may identify ligaments that are over-stressed within the joint, such as when a joint segment is more rigid than expected. In some examples, the computing system tracks the range of motion of the joint during surgery. In some examples, fiducial markers are attached to various points on the leg and foot of the patient. A 3D spatial camera (e.g., a camera with multiple image sensors, optical sensors, and/or laser sensors that captures depth information to construct a 3D image, etc.) track the fiducial markers to provide 3D information about, for example, the kinematic axes of the ankle joint. Additionally, or alternatively, accelerometers and/or other spatial sensors may be attached at various location of the leg and foot to provide spatial feedback about mobility of the ankle joint. For example, using the interoperative tracking, the kinematic flexion axis may be evaluated during surgery to intraoperatively evaluate a surgical intervention. Based on the interoperative feedback, the computing system may dynamically adjust the surgical plan and provide information to the surgeon using the mixed reality system. In some examples, the interoperative data may be collected by the computing system and used to adjust the tasks or functions of interoperative robotics devices, modify interoperative navigation tools, adjust one or more parameters of smart instrumentations, or adjust any other tools employed by the clinician to complete the surgery for the patient. In some examples, these robotics, navigation tools, smart instrumentation, or any other devices may provide information to the computing system intraoperatively, such as during the procedure, and the computing system may modify the procedure responsive to any or all of the received information relating to the patient and/or the procedure. In one example, instrumentation may adjust the type of data or frequency of data collected in response to intraoperatively collected data. In some examples, robotic arms or other devices may turn on or off, or adjust the pre-programmed tasks, in response to intraoperatively collected data for the patient. In this example, the system or aspects of the surgical system can respond to newly collected data during the procedure that may change the surgical plan or parts of the surgical plan.

The techniques described herein improve planning and execution of dynamic joint replacement surgeries, such as total ankle replacement surgery. Using dynamic ankle analysis, stresses within the implant and the bone-implant interface may be minimized. These stress reductions may reduce implant-bearing-surface wear, wear-debris generation, and reduce implant loosening from the bone.

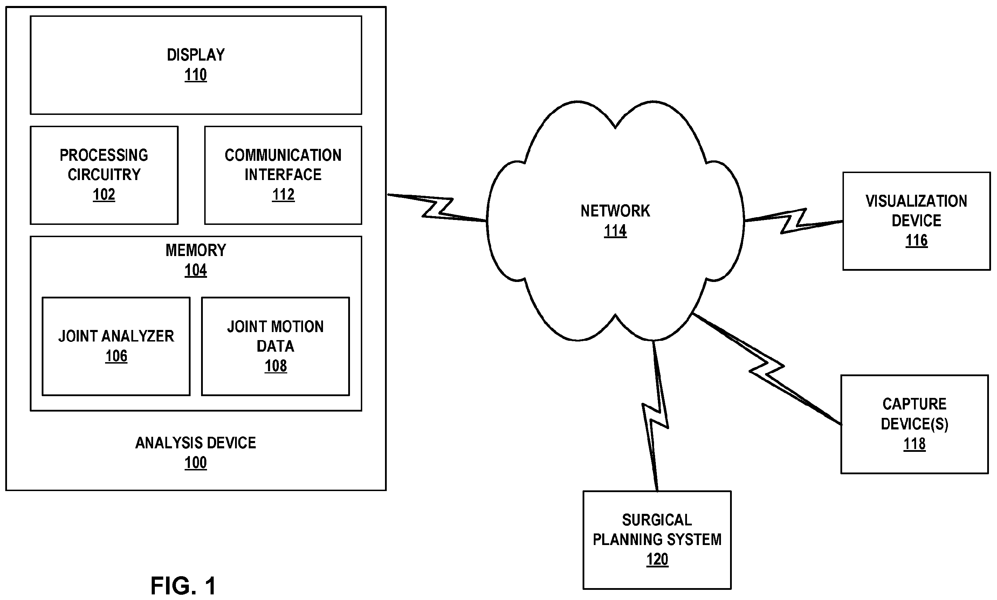

FIG. 1 is a block diagram illustrating an example computing device that may be used to implement the techniques of this disclosure. As shown in the example of FIG. 1, analysis device 100 is an example of a computing device configured to perform one or more example techniques described in this disclosure.

Analysis device 100 may include various types of computing devices, such as server computers, personal computers, smartphones, laptop computers, and other types of computing devices. Analysis device 100 includes processing circuitry 102, memory 104, and display 110. Display 110 is optional, such as in examples where device 100 is a server computer.

Examples of processing circuitry 102 include one or more microprocessors, digital signal processors (DSPs), application specific integrated circuits (ASICs), field programmable gate arrays (FPGAs), discrete logic, software, hardware, firmware or any combinations thereof. In general, processing circuitry 102 may be implemented as fixed-function circuits, programmable circuits, or a combination thereof. Fixed-function circuits refer to circuits that provide particular functionality and are preset on the operations that can be performed. Programmable circuits refer to circuits that can programmed to perform various tasks and provide flexible functionality in the operations that can be performed. For instance, programmable circuits may execute software or firmware that cause the programmable circuits to operate in the manner defined by instructions of the software or firmware. Fixed-function circuits may execute software instructions (e.g., to receive parameters or output parameters), but the types of operations that the fixed-function circuits perform are generally immutable. In some examples, the one or more of the units may be distinct circuit blocks (fixed-function or programmable), and in some examples, the one or more units may be integrated circuits.

Processing circuitry 102 may include arithmetic logic units (ALUs), elementary function units (EFUs), digital circuits, analog circuits, and/or programmable cores, formed from programmable circuits. In examples where the operations of processing circuitry 102 are performed using software executed by the programmable circuits, memory 104 may store the object code of the software that processing circuitry 102 receives and executes, or another memory within processing circuitry 102 (not shown) may store such instructions. Examples of the software include software designed for surgical planning.

Memory 104 may be formed by any of a variety of memory devices, such as dynamic random access memory (DRAM), including synchronous DRAM (SDRAM), magnetoresistive RAM (MRAM), resistive RAM (RRAM), or other types of memory devices. Examples of display 110 include a liquid crystal display (LCD), a plasma display, an organic light emitting diode (OLED) display, or another type of display device.

In the illustrated example, analysis device 100 includes communication interface 112. Communication interface 112 facilitates, for example, analysis device 100 transmitting data and instructions to and receiving data and instructions from visualization device 116 via network 114. For example, after determining impingements and/or ranges of motion about various axes (sometimes referred to as "current joint state data"), using techniques described in this disclosure, communication interface 112 may output the current joint state data to visualization device 116 via network 114. A surgeon may then view a graphical representation of the current joint state data with visualization device 116. For example, in a mixed reality system, the graphical representations of the current joint state data may be displayed on a lens/screen such that the graphical representations of the current joint state data is overlaid on top of the joint of interest as seen by eyes of the surgeon through the lens/screen. In another example, visualization device 116 may include a touch sensitive or non-touch sensitive display. In the illustrated example, communication interface 112 facilitates receiving joint motion data 108 from one or more capture devices 118 (e.g., gait lab systems, image capture systems, image preprocessing systems, medical imaging systems, etc.) via network 114. For example, analysis device 100 may receive joint motion data 108 from an image preprocessing system that performs image recognition techniques to identify positions of bones in CT images of an ankle in different positions. In the illustrated example, communication interface 112 facilitates sending current joint state data to visualization device 116. Additionally, communication interface 112 facilitates sending date indicative of a desired joint state (e.g., a target state of the joint after surgical intervention, etc.) to visualization device 116.

Communication interface 112 may be hardware circuitry that enables device 100 to communicate (e.g., wirelessly or using wires) to other computing systems and devices, such as visualization device 116. Network 114 may include various types of communication networks including one or more wide-area networks, such as the Internet, local area networks, and so on. In some examples, network 114 may include wired and/or wireless communication links.

Visualization device 116 may utilize various visualization techniques to display image content to a surgeon. Visualization device 116 may be a mixed reality (MR) visualization device, virtual reality (VR) visualization device, holographic projector, or other device for presenting extended reality (XR) visualizations. In some examples, visualization device 116 may be a Microsoft HOLOLENS^{™} headset, available from Microsoft Corporation, of Redmond, Washington, USA, or a similar device, such as, for example, a similar MR visualization device that includes waveguides. The HOLOLENS ^{™} device can be used to present 3D virtual objects via holographic lenses, or waveguides, while permitting a user to view actual objects in a real-world scene, i.e., in a real-world environment, through the holographic lenses.

Capture device(s) 118 may use various techniques to capture visual and/or analytical data regarding motion of the joint of interest. In some examples, capture devices 118 include one or more medical imaging devices (e.g., computed tomography (CT) imaging systems, magnetic resonance imaging (MRI) systems) that capture images of tissue such as bones related to the joint of interest in various positions about axis of motion of the joint while loaded (e.g., weight bearing) and unloaded (e.g., not weight bearing). For example, capture device 118 may be an imaging system capable of capturing an ankle joint in a dorsiflexion position, a neutral position, and a plantarflexion position while loaded from the patient's own weight and/or an artificial force as a surrogate for the patient's weight. Alternatively or additionally, in some examples, capture devices 118 include instrumentation (e.g., a motion capture system, visual light and/or infrared cameras, a force plate, pressure sensors, force sensors, and/or accelerometers, etc.) to perform and software to process data from a gait lab. Capture devices 118 may be configured to generate joint motion data that identifies relationships between bones and/or appendages related to the joint of interest to facilitate determining motion about one or more axes of the joint.

Surgical planning system 120 may be configured to generate a virtual surgical plan customized to transform a current kinematic state of a joint to a desired kinematic state through surgical intervention, such as an implant and/or modification of soft tissue (e.g., ligaments, tendons, etc.). In some examples, surgical planning system 120 may generate the virtual surgical plan to include steps and/or processes to prepare anatomy for attachment of an implant or attachment of the implant to the anatomy. For example, the virtual surgical plan may include one or more processes to prepare a tibia bone and/or a talus bone and/or one or more processes to attach an implant to the tibia bone and/or an implant to the talus bone. In some examples, the surgical intervention is a total ankle arthroplasty. The virtual surgical plan may include a 3D virtual model corresponding to the joint of interest of the particular patient and a 3D model of an implant component matched to the particular patient to perform the surgical intervention. In the illustrated example, analysis device 110 and surgical planning device 120 are separated devices communicatively coupled by network 114. However, in some examples, analysis device 110 and surgical planning device 120 may be embodied by the same device.

Surgical planning system 120 may utilize visualization tools that are available to utilize patient image data to generate three-dimensional models of bone contours to facilitate preoperative planning for joint repairs. These tools allow surgeons to design and/or select surgical guides and implant components that closely match the patient's anatomy. These tools can improve surgical outcomes by customizing a surgical plan for each patient. An example of such a visualization tool for shoulder repairs is the BLUEPRINT ^{™} system available from Wright Medical Technology, Inc. The BLUEPRINT ^{™} system provides the surgeon with two-dimensional planar views of the bone repair region as well as a three-dimensional virtual model of the repair region. The surgeon can use the BLUEPRINT ^{™} system to select, design or modify appropriate implant components, determine how best to position and orient the implant components and how to shape the surface of the bone to receive the components, and design, select or modify surgical guide tool(s) or instruments to carry out the surgical plan. The information generated by the BLUEPRINT ^{™} system is compiled in a preoperative surgical plan for the patient that is stored in a database at an appropriate location (e.g., on a server in a wide area network, a local area network, or a global network) where it can be accessed by the surgeon or other care provider, including before and during the actual surgery. In some examples, the virtual surgical plan specifies one or more of (a) a size, a shape, and/or a placement of an implant, (b) a modification to one or more regions of soft tissue (e.g., tendons, ligaments, etc.) of the patient, and/or (c) a correction to at least one bone associated with a bony impingement.

Surgical planning system 120 may provide the virtual surgical plan to visualization device 116 to present a user interface to a surgeon. The user interface may present details of the virtual surgical plan for a particular patient. For instance, the details of the virtual surgical plan may include a 3D virtual model of a joint of interest of the particular patient. The user interface is visually perceptible to the user when the user is using visualization device 116. For instance, in one example, a screen of visualization device 116 may display real-world images and the user interface on a screen. In some examples, visualization device 116 may project virtual, holographic images onto see-through holographic lenses and also permit a user to see real-world objects of a real-world environment through the lenses. In other words, visualization device 116 may comprise one or more see-through holographic lenses and one or more display devices that present imagery to the user via the holographic lenses to present the user interface to the user.

As illustrated, memory 104 stores data representative of a joint analyzer 106 and patient specific data representative of motion of a joint of interest (sometimes referred to as "joint motion data 108"). Joint analyzer 106 performs dynamic analysis on the joint motion data 108 to determine current kinematic state of the joint of interest. For example, transformation generator 106 performs dynamic analysis on joint motion data 108 containing ankle motion data to characterized the current properties (such as, the kinematic flexion axis, the kinematic inversion/eversion axis, the kinematic abduction/adduction axis, and/or the soft tissue constraints, etc.) of the ankle of a patient.

In some examples, joint analyzer 106, using joint motion data 108, analyzes the relative transform between bones that form the joint of interest to determine the translational and rotational flexibility of the joint. For example, joint analyzer 106 may calculate a relative transformation of the tibia and talus from joint motion data 108 to compute the motion of the talus relative to the tibia to determine the motion of the ankle joint about one or more of the kinematic flexion axis, the kinematic inversion/eversion axis, or the kinematic abduction/adduction axis. Alternatively or additionally, in some examples, joint analyzer 106 may use joint motion data 108 to morph a standardized, three-dimensional computational model of the joint of interest into a patient-specific computational model of the joint. This initial standardized model may be one of a plurality of standards models and may be selected as the initial model based on demographic and/or physical characteristics of the patient. The initial model may be morphed to account for the size, shape, and/or relative alignment of the relevant bones (e.g., the tibia and the talus) and/or attachment points of relevant soft tissue (e.g., ligaments and tendons, etc.) specific to the patient. Joint motion data 108 obtained through the clinical gait lab and/or the multiple image analysis may be used to morph the three-dimensional computational model

The patient-specific computational model may be run to analytically determine the motion of the joint. The patient-specific computational model may also determine range of motion constraints to include in data sent to surgical planning system 120. For example, the patient-specific computational model may determine that an ankle joint exhibits insufficient plantarflexion, excessive coronal tilt during walking (e.g., compared to the statistical average of a patient with similar demographic and physical attributes), and/or an exaggerated adjacent joint compensation. The patient-specific computation model may determine impingements to be highlighted that prevent the joint moving on a normal kinematic axis, such as bony impingements caused by ankle gutters or tendons that are too tight. These technique facilitate identifying a current joint kinematic axes of the patient to provide data from which to generate the surgery plan (e.g., by surgical planning system 120).

In some examples, analysis device 100, visualization device 116, capture device(s) 118, and/or surgical planning system 120 are communicatively coupled during an operation to provide interoperative tracking of joint mobility and feedback to a surgeon. In some examples, capture device(s) 118 may capture images and/or inertial sensor data during a surgery. Analysis device 100 analyzes the interoperative joint motion data to determine a current j oint motion status to determine a difference between the current j oint motion status and the target joint motion status determined before surgery. In some examples, analysis device 100 transmits the interoperative joint motion data to surgical planning system 120 to change the surgical plan or to provide more detailed guidance. Subsequently, surgical planning system 120 may provide updated guidance to visualization device 116. For example, images capture during surgery may detect a bony impingement at a certain flexion angle that was not detected in preoperative images (e.g., due to the size of the bony impingement and/or the clarity of preoperative images, etc.). Visualization device 118 may highlight the detected impingement and, in some examples, provide visual guidance of suggested interventions to correct it. For example, visualization device 118 may highlight the intraoperatively detected bony impingement and, in some examples, provide visual guidance of suggested modifications to the bone to correct the impingement.

In some examples, analysis device 100 tracks the range of motion of the joint during surgery. In some examples, fiducial markers and/or inertial sensors (e.g., accelerometers, gyroscopes, etc.) are attached to various points on the relevant appendages (e.g., the leg and the foot, etc.) of the patient. In some such examples, a 3D spatial camera (e.g., a camera with multiple image sensors, optical sensors, and/or laser sensors that captures depth information to construct a 3D image, etc.) track the fiducial markers to provide 3D information regarding the interoperative (e.g., intermediate or updated) kinematic axes of the joint of interest. Additionally or alternatively, accelerometers and/or other spatial sensors may be attached at various location of the leg and foot to provide spatial feedback about mobility of the ankle joint. After a surgical intervention is performed, the joint may be manipulated (e.g., manually, by a jig designed to interoperative manipulate the joint in a controlled manner, etc.). The movement may be tracked by capture devices 118. For example, using the interoperative tracking, the kinematic flexion axis may be evaluated during surgery to intraoperatively evaluate a surgical intervention. Analysis device 100 determines wherein the intraoperatively detected intermediate kinematic axes of the joint of interest aligns with the target kinematic axes of the joint of interest. This data is then provided to surgical planning system 120 to update, when necessary, the virtual surgical plan. For example, upon receiving updated analysis data from analysis device 100, surgical planning system 120 may alter the virtual surgical plan when the intermediate kinematic axes of the joint of interest aligns is substantially different than the target kinematic axes of the joint of interest. In some examples, based on the interoperative feedback, surgical planning system 120 dynamically adjusts the virtual surgical plan and provides feedback to the surgeon using the mixed reality system via the visualization device 116.

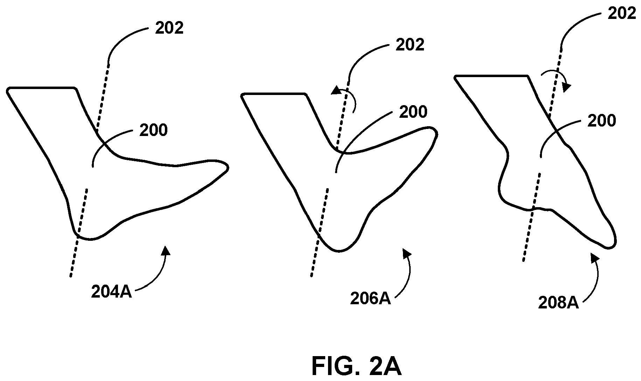

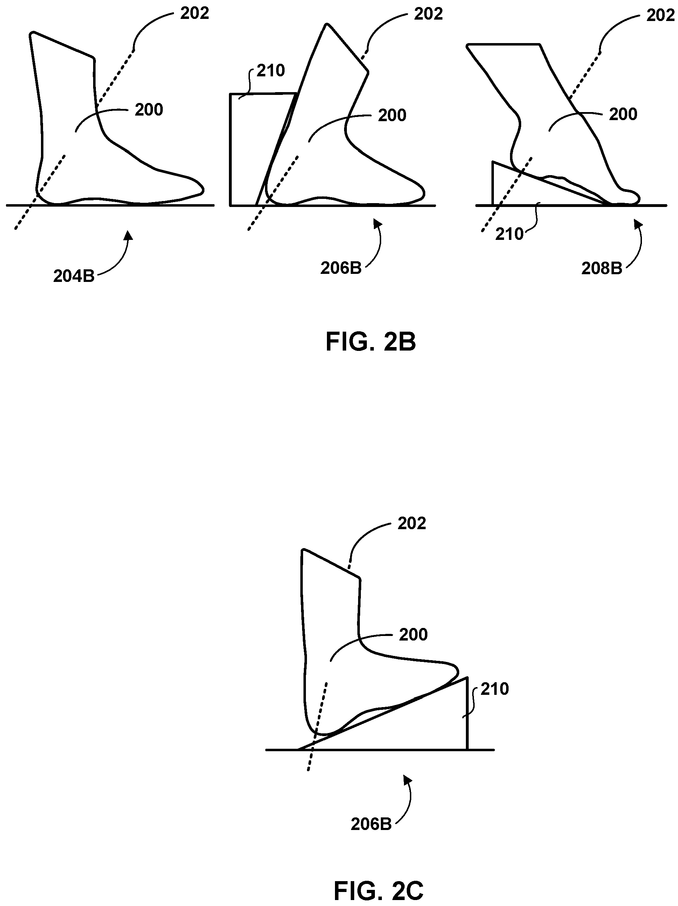

FIGS. 2A, 2B, and 2Cillustrate example positions in which to obtain images of an ankle 200 about a kinematic flexion axis 202 for dynamic ankle analysis in accordance with the teachings of this disclosure. In the illustrated examples, ankle 200 is positioned in three positions about kinematic flexion axis 202. In FIG. 2A, ankle 200 is in a neutral position 204, a dorsiflexion position 206, and a plantarflexion position 208. As described above, capture device 118 captures data (e.g., images, motion capture, etc.) of ankle 200 in these position to generate joint motion data 108. Based of joint motion data 108 of ankle 200 in neutral position 204A, dorsiflexion position 206A, and plantarflexion position 208A, joint analyzer 106 determines the motion of ankle 200 about kinematic flexion axis 202. In the illustrated example of FIG. 2A, capture device 118 captures data of ankle 200 in neutral position 204, dorsiflexion position 206, and plantarflexion position 208. Capture device 118 may capture data of ankle 200 in any number of position about any number of axes (e.g., a kinematic abduction/adduction axis, kinematic flexion axis 202, a kinematic inversion/eversion axis, etc.). In the example of FIG. 2B, ankle 200 is positioned in three positions about kinematic flexion axis 202 (e.g., neutral position 204B, dorsiflexion position 206B, and plantarflexion position 208B) under a loaded condition. Under the loaded condition, weight of the patient is applied to ankle 200 to determine the initial kinematic axis while under a load of the patient. Capture device 118 captures joint motions data 108 of ankle 200 while in the loaded condition. In some examples, capture device 118 includes structures 210 to brace ankle 200 in place while in the loaded condition to facilitate capturing images. In the illustrated example of FIG. 2C, ankle 200 is positioned in dorsiflexion position 206B under the loaded condition using a different configuration of structures 210 to brace ankle 200 in place.

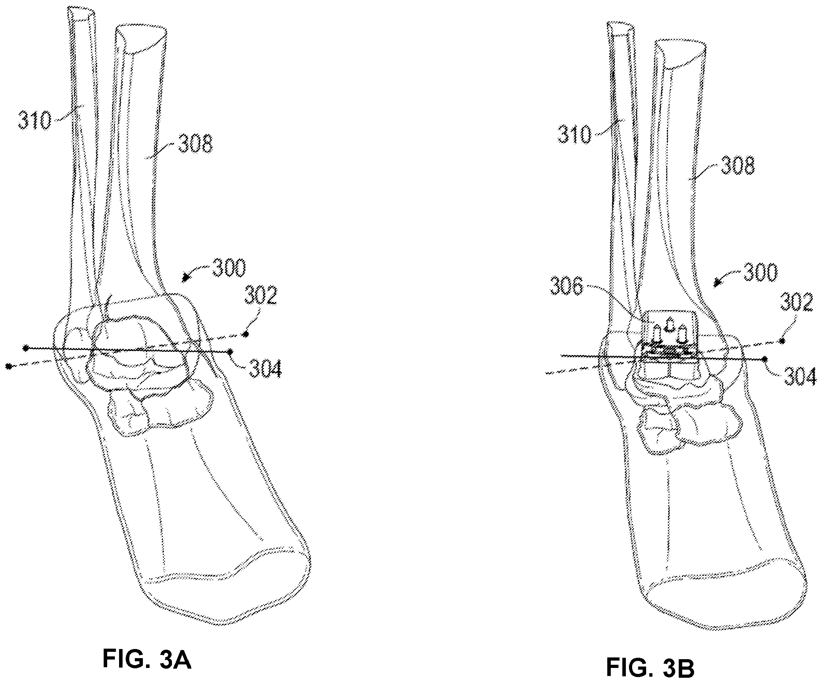

FIGS. 3A and 3B illustrate an example joint 300 depicting an initial kinematic axis 302 and a target kinematic axis 304. As described above, analysis device 100 determines, based on joint motion data 108, initial kinematic axis 302. For example, analysis device 100 may calculate a relative transformation of the tibia and talus from CT images of an ankle (e.g., ankle 200) in multiple positions (e.g., neutral position 204, dorsiflexion position 206, and plantarflexion position 208) to compute the motion of the talus relative to the tibia to determine the kinematic flexion axis. Analysis device 100 may be configured to determine target kinematic axis 304 based on, for example, physical and/or demographic features of the patient. For example, analysis device 100 may determine that the initial kinematic flexion axis 302 is caused by diseased bone and/or cartilage, soft-tissue restraints, and/or bony impingements. Based on initial kinematic axis 302 and target kinematic axis 304, surgical planning system 120 selects one or more surgical interventions. In some examples, surgical planning system 120 selects an implant 306 to transition from initial kinematic axis 302 and target kinematic axis 304. In some examples, to transition from initial kinematic axis 302 and target kinematic axis 304, surgical planning system 120 may specify adjustments to one or more soft tissue impingements and/or bony impingements. FIG. 3A illustrates joint 300 in an initial aligned position along initial kinematic axis 302 where implant 306 has not been installed and impingements have not been addressed. FIG. 3B illustrates joint 300 in a target aligned position along target kinematic axis 304 where implant 306 has been installed and impingements have been addressed. In the illustrated examples, to transition joint 300 to target kinematic axis 304 from initial kinematic axis 302, implant 306 may be selected such that (i) coronal plane (e.g., axis of rotation around the intersecting point between malleoli and the long axis of the tibia in the frontal plane) is adjusted 11.2 degrees more varsus, (ii) axial plant is 3.9 degrees more external, (iii) M-L axis (e.g., vertical axis for abduction and adduction, etc.) is translated 2.0 millimeters (mm) more lateral, (iv) A-P axis (e.g., longitudinal axis for inversion/eversion) is translated 11mm more anterior, and (v) the joint line elevation change is 2.4 mm proximal on the lateral side and 3 mm distal on the medial side. To show implant 306 more fully, tibia 308 and fibula 310 in FIGS. 3A and 3B are transparent.

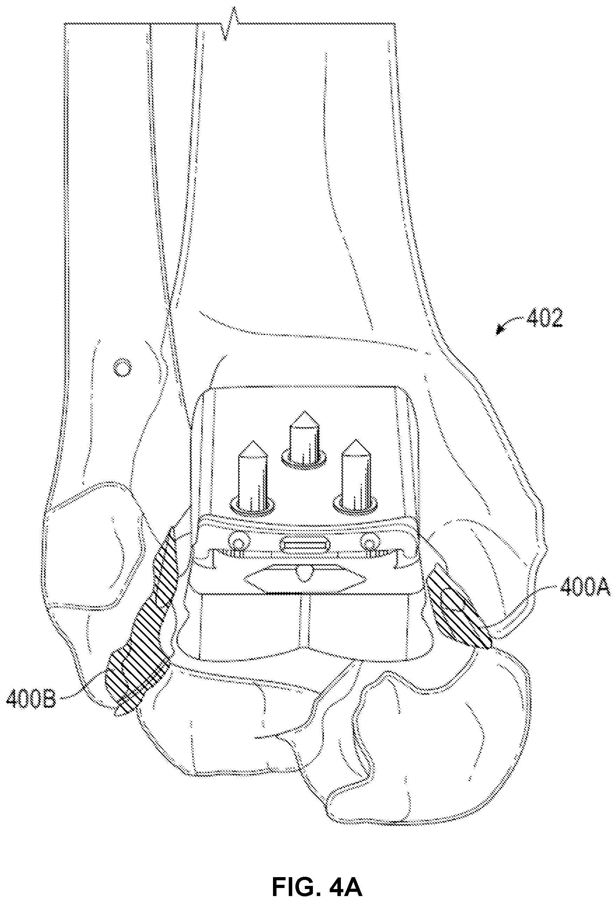

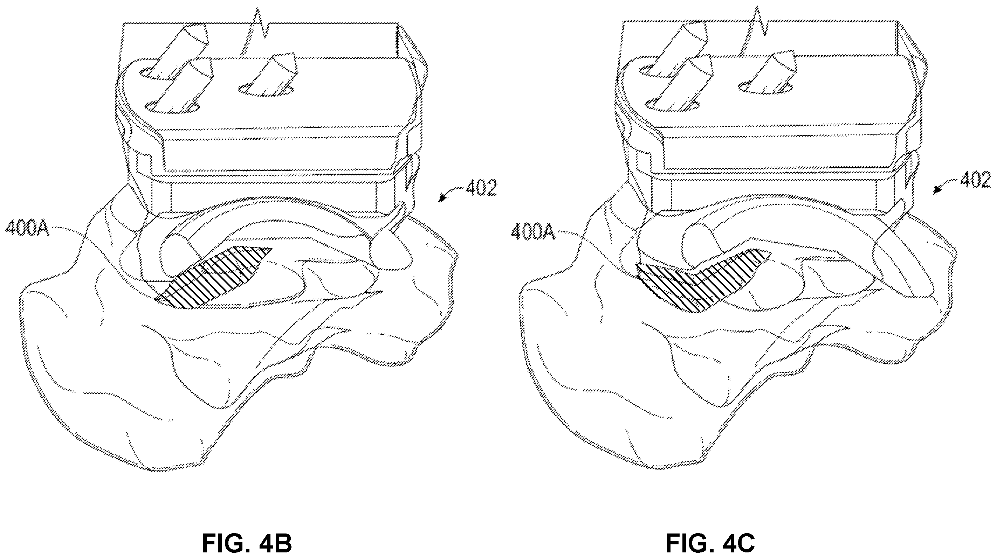

FIG. 4A, 4B, and 4C illustrate example visual representations 400A and 400B (shown with cross-hatchings) of impingements to movement of a joint 402. As described above, analysis device 100 may be configured to determine impingements to movement based on an analysis of joint motion data 108. Analysis device 100 may detect impingements preoperatively and/or intraoperatively. For example, analysis device 100 may detect impingements for visualization from joint motion data 108 derived from images captured before the operation. As another example, analysis device 100 may detect impingements for visualization from joint motion data 108 derived from tracking the range of motion of the joint during surgery. Analysis device 100 provides data regarding impingements to movement to visualization device 116 and/or surgical planning system 120. For example, analysis device 100 may detect impingements to movement, surgical planning system 120 may generate visual representations 400 of impingements to movement, and visualization device 116 may display visual representations 400A and 400B. FIG. 4C illustrates ankle 402 from a coronal direction depicting visual representations 400A and 400B of the zones of impingement. Impingements to movement may be different as joint 402 moves in its expected range of motion. In FIG. 4B, visual representation 400A is display when joint 402 (or, in some examples, the virtual representation joint 402) in in a neutral position (e.g., neutral positions 204A and 204B of FIGS 2A, 2B, and 2C, etc.). As illustrated in FIG. 4C, visual representations (e.g., visual representation 400A) may change characteristics (e.g., size, location, area, etc.) as joint 402 rotates as the angle between the foot and leg changes. In FIG. 4C, joint 402 is in 10 degrees of dorsiflexion. As such, as joint 402 moves, different visual representations of impingements to movement may be displayed to correspond to the current position of joint 402.

In some examples, visualization device 116 may display visual representations 400A and 400B via a mixed reality system in which visual representations 400A and 400B are presented as virtual objects. In such a mixed reality system, a user may see real, physical objects (e.g., joint 402) through a transparent display and virtual objects (e.g., visual representations 400A and 400B) projected onto the transparent display. In such examples, mixed reality system may perform a registration process to register (e.g., align) the virtual object to corresponding objects in physical space. In such a manner, visualization device 118 may highlight the detected impingement and, in some examples, provide visual guidance of suggested interventions to correct it. For example, visualization device 118 may highlight a soft tissue impingement and, in some examples, provide visual guidance of suggested modifications to correct the impingement. Suggested modifications may include, for example, implant placement and alignment, bone modifications, and/or soft tissue modifications.

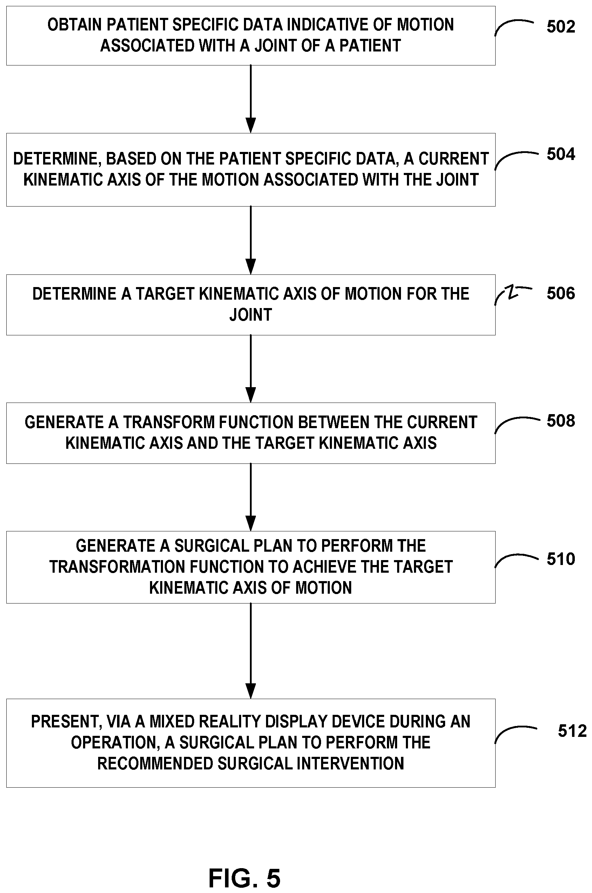

FIG. 5 is a flowchart of an example method to generate a surgical plan based on a dynamic joint analysis in accordance with the teachings of this disclosure. The example technique may be performed by analysis device 100, capture device(s) 118, and/or surgical planning system 120. Initially, capture device(s) 118 obtain patient specific data indicative of motion (e.g., joint motion data 108) associated with a joint (e.g., ankle 200) of a patient (502). For example, one or more capture devices 118 may capture images of ankle 200 in different positions. In another example, one or more capture devices 118 may perform a gait analysis. Processing circuitry 102 of analysis device 100 determines, based on the patient specific data (e.g., joint motion data 108), a current kinematic axis of the motion associated with the joint (504). For example, processing circuitry 102 of analysis device 100 may calculate a relative transformation of the tibia and talus from joint motion data 108 of an ankle (e.g., ankle 200) in multiple positions (e.g., neutral position 204, dorsiflexion position 206, and plantarflexion position 208) to compute the motion of the talus relative to the tibia to determine the kinematic flexion axis. In another example, analysis device 100 may calculate a relative transformation of a humerus relative to a scapula in multiple positions to determine a mediolateral axis of motion of a shoulder.

Processing circuitry 102 of analysis device 100 then determines a target kinematic axis of motion for the joint based on, for example, physical and/or demographic features of the patient (506). Surgical planning system 120 may generate a transform function between the current kinematic axis and the target kinematic axis (508). The transform function may include one or more interventions to modify the joint to achieve the target kinematic axis. For example, the transforms function may include steps to install an implant of a certain size and/or shape, modify soft tissues, and/or modify bone. Surgical planning system 120 generates a recommended surgical plan to perform the transformation function to achieve the target kinematic axis of motion (510). In some examples, the surgical plan includes an ordered list of steps, with accompanying visual data, to implement the transform function. Visualization device 110 may present (e.g., via a mixed reality display device), during an operation, the surgical plan to perform the recommended surgical interventions (512).

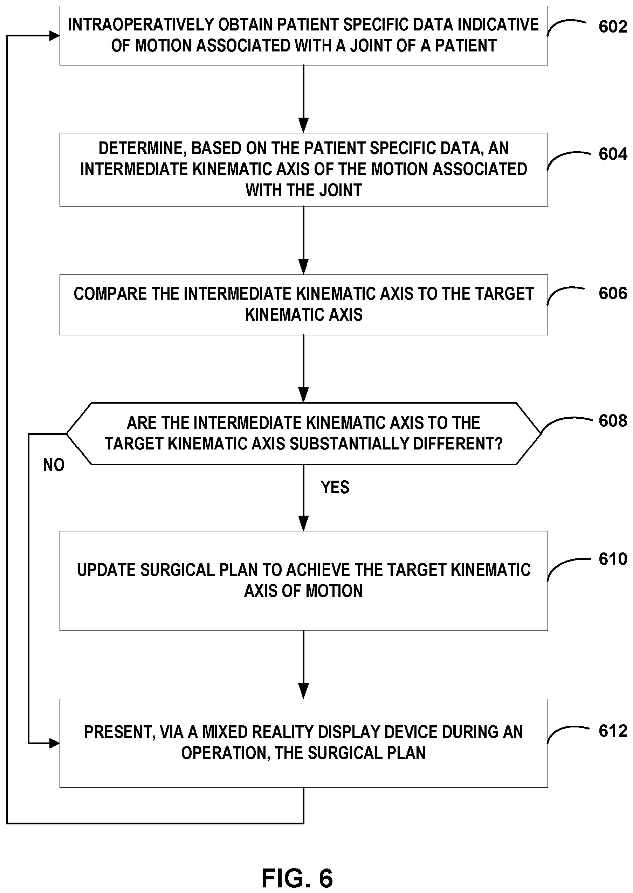

FIG. 6 is a flowchart of an example method to intraoperatively create or change a surgical plan based on a dynamic joint analysis in accordance with the teachings of this disclosure. Capture device 118 intraoperatively obtains patient specific data indicative of motion associated with a joint of a patient (602). In some examples, fiducial markers and/or inertial sensors are attached to various points on the relevant appendages of the patient. In some such examples, data capture devices (e.g., cameras, accelerometers, etc.) measure movement of the relevant appendages to provide interoperative joint motion data 108. In some examples, some of these sensors may be referred to as smart instrumentation when they are configured to generate and transmit one or more types of data indicative of the use of a tool during a procedure and/or a parameter representative of a patient state or change. These smart instruments may transmit data that is viewable by the physician as part of the intraoperative patient specific data and/or used by a computer system to adjust and control one or more aspects of the procedure. In some examples, one or more interoperative robotics devices or navigation tools may incorporate the patient specific data obtained interoperatively to change a surgical plan as described below.

Analysis device 100 then determines, based on interoperative joint motion data 108, an intermediate kinematic axis of the motion associated with the joint (604). Analysis device 100 compares the intermediate kinematic axis to the target kinematic axis (606). If the intermediate kinematic axis is substantially different from the target kinematic axis ("YES" branch of block 608), analysis device 100 sends the updated joint motion data 108 to surgical planning system 120. The surgical planning system 120 then updates surgical plan to achieve the target kinematic axis of motion (610). For example, surgical planning system 120 may recommend selecting a different implant component or implant location, adjusting soft tissue, or further modify a bony impingement. Visualization device 116 presents (e.g., via a mixed reality display device), during an operation, the updated surgical plan (612). When the intermediate kinematic axis is substantially different from the target kinematic axis (NO at 608), Visualization device 116 presents (e.g., via a mixed reality display device), during an operation, the surgical plan (612).

In some examples, the interoperative data may be collected by the computing system (e.g., from smart instrumentation and/or other obtained patient specific data), and the computing system may execute the updated surgical plan to adjust the tasks or functions of interoperative robotics devices, modify interoperative navigation tools, adjust one or more parameters of other smart instrumentations, or adjust any other tools employed by the clinician to complete the surgery for the patient. In this manner, updating the surgical plan may include automatically adjusting one or more parameters of a robotic device in order to achieve the target kinematic axis of motion based on the updated patient specific data obtained intraoperatively. In some examples, any of capture devices 118 may be used interoperatively to provide patient specific data that can be utilized by the system to update a surgical plan or otherwise adjust the procedure as needed based on newly obtained information.

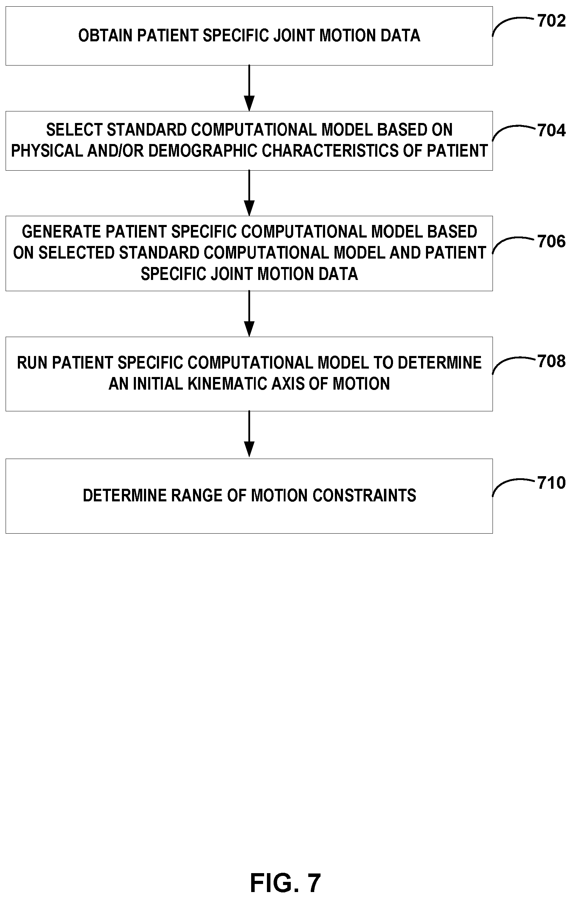

FIG. 7 is a flowchart of an example method to generate a patient specific computational model to determine an initial kinematic axis of a joint in accordance with the teachings of this disclosure.

Initially, capture device 118 obtains patient specific joint motion data 108 (702). For example, capture device 118 may obtain joint motion data 108 by tracking motion (e.g., via fiducial markers, inertial sensors, force sensors, and/or pressure sensors, etc.) of a patient from which characteristics of bone and soft tissue can be derived (e.g., by processing circuitry 102 of analysis device 100). Additionally or alternatively, in some examples, capture device 118 may obtain joint motion data 108 by analyzing multiple static images of the joint of interest in different positions from which the relative motion of the bones related to the joint can be determined (e.g., by processing circuitry 102 of analysis device 100). Processing circuitry 102 of analysis device 100 selects a standard computational model from a plurality of standard computational models based on physical and/or demographic characteristics of patient (704). For example, processing circuitry 102 may select the standard computational model from a plurality of standard computational models based on different characteristics. These computational models may be based on characteristics of a person or persons from which the model was determined, such as patient height, weight, age, sex, race, disease, condition, trauma, or any other characteristic. Processing circuitry 102 may select the standard computational model based on a person or persons with characteristics that most closely match the patient. Processing circuitry 102 of analysis device 100 generates a patient specific computational model based on the selected standard computational model and patient specific joint motion data 108 (706). For example, the size and/or shape of the bones related to the joint of interest and/or soft tissue connection points may be changed (e.g., morphed, stretched, etc.) in the selected computational model based on the patient specific joint motion data 108 such that the behavior of adjusted computational model substantially mimics the behavior of the joints of interest of the patient. This adjustment may result in a standard computational model that processing circuitry 102 morphs to fit the patient-specific measurements and generate a patient-specific computational model. As another example, the patient specific computational model may be generated through computational statistical methods, such as statistical shape modeling (SSM). Example techniques to perform statistical shape modeling are described in U.S. Provisional Application No. 62/826,362, "Pre-Morbid Characterization Of Anatomical Object Using Statistical Shape Modeling (SSM)," filed March 29, 2019; and in U.S. Provisional Application No. 63/021,337, "Pre-Morbid Characterization Of Anatomical Object Using Orthopedic Anatomy Segmentation Using Hybrid Statistical Shape Modeling (SSM)," filed May 7, 2020. As another example, the selected computational model may be modified to include bony and/or soft-tissue impingements. Analysis device 100 runs patient specific computational model to determine an initial kinematic axis of motion (708). In some examples, processing circuitry 102 of analysis device 100 determines range of motion constraints (e.g., soft tissue constraints, diseased bone and/or cartilage restraints, etc.) according to the patient specific computational model (710). Processing circuitry 102 of analysis device 100 uses joint motion data 108 derived from the joint computation model to determine a current range of motion status and a target joint motion status for the joint of the patient. Subsequently, surgical planning system 120 may generate visual representations for the identified range of motion constraints, the current range of motion status, and/or a target joint motion status, which may then be displayed by visualization device 116. In some examples, surgical planning system 120 may provide recommendations of modifications to correct the impingement (e.g., implant placement and alignment, bone modifications, and/or soft tissue modifications, etc.).

While the techniques been disclosed with respect to a limited number of examples, those skilled in the art, having the benefit of this disclosure, will appreciate numerous modifications and variations there from. For instance, it is contemplated that any reasonable combination of the described examples may be performed.

It is to be recognized that depending on the example, certain acts or events of any of the techniques described herein can be performed in a different sequence, may be added, merged, or left out altogether (e.g., not all described acts or events are necessary for the practice of the techniques). Moreover, in certain examples, acts or events may be performed concurrently, e.g., through multi-threaded processing, interrupt processing, or multiple processors, rather than sequentially.

In one or more examples, the functions described may be implemented in hardware, software, firmware, or any combination thereof. If implemented in software, the functions may be stored on or transmitted over as one or more instructions or code on a computer-readable medium and executed by a hardware-based processing unit. Computer-readable media may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media which is non-transitory or (2) a communication medium such as a signal or carrier wave. Data storage media may be any available media that can be accessed by one or more computers or one or more processors to retrieve instructions, code and/or data structures for implementation of the techniques described in this disclosure. A computer program product may include a computer-readable medium.

By way of example, and not limitation, such computer-readable storage media can comprise RAM, ROM, EEPROM, CD-ROM or other optical disk storage, magnetic disk storage, or other magnetic storage devices, flash memory, or any other medium that can be used to store desired program code in the form of instructions or data structures and that can be accessed by a computer. Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. It should be understood, however, that computer-readable storage media and data storage media do not include connections, carrier waves, signals, or other transitory media, but are instead directed to non-transitory, tangible storage media. Disk and disc, as used herein, includes compact disc (CD), laser disc, optical disc, digital versatile disc (DVD), floppy disk and Blu-ray disc, where disks usually reproduce data magnetically, while discs reproduce data optically with lasers. Combinations of the above should also be included within the scope of computer-readable media.

Operations described in this disclosure may be performed by one or more processors, which may be implemented as fixed-function processing circuits, programmable circuits, or combinations thereof, such as one or more digital signal processors (DSPs), general purpose microprocessors, application specific integrated circuits (ASICs), field programmable gate arrays (FPGAs), or other equivalent integrated or discrete logic circuitry. Fixed-function circuits refer to circuits that provide particular functionality and are preset on the operations that can be performed. Programmable circuits refer to circuits that can programmed to perform various tasks and provide flexible functionality in the operations that can be performed. For instance, programmable circuits may execute instructions specified by software or firmware that cause the programmable circuits to operate in the manner defined by instructions of the software or firmware. Fixed-function circuits may execute software instructions (e.g., to receive parameters or output parameters), but the types of operations that the fixed-function circuits perform are generally immutable. Accordingly, the terms "processor" and "processing circuity," as used herein may refer to any of the foregoing structures or any other structure suitable for implementation of the techniques described herein.

## Claims

1. A system comprising:
processing circuity (102) configured to:
receive patient specific data (108) indicative of pre-operative motion associated with an ankle (200) of a patient;
determine, based on the patient specific data, a current kinematic axis (302) of the motion associated with the ankle;
determine a target kinematic axis (304) of motion for the ankle, the target kinematic axis being different than the current kinematic axis;
generate a transform function between the current kinematic axis and the target kinematic axis; and
generate, based on the transform function, a recommended surgical intervention that adjusts tissue associated with the ankle to achieve the target kinematic axis of motion.

2. The system of claim 1, wherein the processing circuity is further configured to:
analyze the pre-operative motion associated with the ankle of the patient from movement data obtained from video and inertial sensor data captured while the patient is moving; and
generate, based on the analysis of the pre-operative motion associated with the ankle, the patient specific data.

3. The system of any of claims 1 or 2, wherein the processing circuity is further configured to generate the patient specific data from a plurality of images of the ankle of the patient in respective different positions.

4. The system of claim 3, wherein each image of the plurality of images of the ankle is captured while the ankle is under load from the patient.

5. The system of claim 3, wherein the respective different positions comprise:
a first position wherein a foot associated with the ankle is in a dorsi-flexion position,
a second position wherein the foot is in a neutral position, and
a third position wherein the foot is in a plantar flexion position.

6. The system of any of claims 3 or 5, wherein to determine the current kinematic axis of motion associated with the ankle, the processing circuity is configured to:
calculate a relative transform of a tibia and a talus in each position of the respective different positions; and
calculate, based on the relative transform, the current kinematic axis of motion associated with the ankle.

7. The system of any of claims 1 through 6, wherein to generate the patient specific data, the processing circuity is configured to modify a standardized three-dimensional foot and ankle model during a gait cycle into a patient-specific model based on a plurality of images of the ankle of the patient.

8. The system of claim 7, wherein the patient-specific model incorporates external loading conditions and muscle forces experienced by the ankle of the patient during the gait cycle.

9. The system of any of claims 1 through 8, wherein to determine the target kinematic axis of motion that is different than the current kinematic axis of motion, the processing circuity is configured to select, based on one or more characteristics of the patient, the target kinematic axis of motion from a database of potential kinematic axes of motion.

10. The system of any of claims 1 through 9, wherein to determine the target kinematic axis of motion that is different than the current kinematic axis of motion, the processing circuity is configured to classify the ankle of the patient as one of hyper-mobile or hypo-mobile.

11. The system of any of claims 1 through 10, wherein to determine the target kinematic axis of motion that is different than the current kinematic axis of motion, the processing circuity is configured to identify that the ankle of the patient exhibits, based the current kinematic axis of motion, at least one of an insufficient plantar flexion, an excessive coronal tilt, or an exaggerated adjacent joint compensation.

12. The system of any of claims 1 through 11, wherein the recommended surgical intervention comprises a surgical plan that specifies at least one of a size, a shape, and a placement of an implant.

13. The system of any of claims 1 through 12, wherein the recommended surgical intervention comprises a surgical plan to modify at least one region of soft tissue of the patient and/or
wherein the recommended surgical intervention comprises a surgical plan to correct at least one bone associated with a bony impingement.

14. The system of any of claims 1 through 13, wherein the processing circuity is further configured to:
while the recommended surgical intervention is being performed, evaluate an intermediate kinematic axis of motion associated with the ankle to determine whether the intermediate kinematic axis of motion aligns with the target kinematic axis of motion;
identify, based on the intermediate kinematic axis of motion, one or more sources of limitations of motion that prevent the intermediate kinematic axis of motion from matching the target kinematic axis of motion; and
alter the recommended surgical intervention based on a difference between the intermediate kinematic axis of motion and the target kinematic axis of motion.

15. The system of any of claims 1 through 14, further comprising a visualization device to present, during an operation, via a mixed-reality headset, a graphical representation of the recommended surgical intervention.

## Patentansprüche

1. System, umfassend:
eine Verarbeitungsschaltlogik (102), die konfiguriert ist zum:
Empfangen von patientenspezifischen Daten (108), die auf eine präoperative Bewegung, die einem Knöchel (200) eines Patienten zugeordnet ist, hinweisen;
Bestimmen einer aktuellen kinematischen Achse (302) der Bewegung, die dem Knöchel zugeordnet ist, auf der Grundlage der patientenspezifischen Daten;
Bestimmen einer kinematischen Zielachse (304) der Bewegung für den Knöchel, wobei sich die kinematische Zielachse von der aktuellen kinematischen Achse unterscheidet;
Erzeugen einer Transformationsfunktion zwischen der aktuellen kinematischen Achse und der kinematischen Zielachse; und
Erzeugen einer empfohlenen chirurgischen Intervention auf der Grundlage der Transformationsfunktion, die das dem Knöchel zugeordnete Gewebe anpasst, zum Erreichen der kinematischen Zielachse der Bewegung.

2. System nach Anspruch 1, wobei die Verarbeitungsschaltlogik ferner konfiguriert ist zum:
Analysieren der präoperativen Bewegung, die dem Knöchel des Patienten zugeordnet ist, anhand von Bewegungsdaten, die aus Video- und Trägheitssensordaten erhalten wurden, die erfasst wurden während sich der Patient bewegt; und
Erzeugen der patientenspezifischen Daten auf der Grundlage der Analyse der präoperativen Bewegung, die dem Knöchel zugeordnet ist.

3. System nach einem der Ansprüche 1 oder 2, wobei die Verarbeitungsschaltung ferner so konfiguriert ist, dass sie die patientenspezifischen Daten aus einer Vielzahl von Bildern des Knöchels des Patienten in jeweils unterschiedlichen Positionen erzeugt.

4. System nach Anspruch 3, wobei jedes Bild aus der Vielzahl von Bildern des Knöchels aufgenommen wird, während der Knöchel unter der Belastung des Patienten steht.

5. System nach Anspruch 3, wobei die jeweiligen verschiedenen Positionen umfassen:
eine erste Position, in der sich ein dem Knöchel zugeordneter Fuß in einer Dorsalflexionsposition befindet,
eine zweite Position, in der sich der Fuß in einer neutralen Position befindet, und
eine dritte Position, bei der sich der Fuß in einer Plantarflexionsposition befindet.

6. System nach einem der Ansprüche 3 oder 5, wobei zum Bestimmen der aktuellen kinematischen Bewegungsachse, die dem Knöchel zugeordnet ist, die Verarbeitungsschaltung konfiguriert ist zum:
Berechnen einer relativen Transformation einer Tibia und eines Talus in jeder Position der jeweiligen unterschiedlichen Positionen; und
Berechnen der aktuellen kinematischen Bewegungsachse, die dem Knöchel zugeordnet ist, auf der Grundlage der relativen Transformation.

7. System nach einem der Ansprüche 1 bis 6, wobei die Verarbeitungsschaltung zum Erzeugen der patientenspezifischen Daten so konfiguriert ist, dass sie ein standardisiertes dreidimensionales Fuß- und Knöchelmodell während eines Gangzyklus auf der Grundlage einer Vielzahl von Bildern des Knöchels des Patienten in ein patientenspezifisches Modell modifiziert.

8. System nach Anspruch 7, wobei das patientenspezifische Modell externe Belastungsbedingungen und Muskelkräfte berücksichtigt, denen der Knöchel des Patienten während des Gangzyklus ausgesetzt ist.

9. System nach einem der Ansprüche 1 bis 8, wobei zum Bestimmen der kinematischen Zielbewegungsachse, die sich von der aktuellen kinematischen Bewegungsachse unterscheidet, die Verarbeitungsschaltung konfiguriert ist zum Auszuwählen der kinematischen Zielbewegungsachse aus einer Datenbank potenzieller kinematischer Bewegungsachsen auf der Grundlage einer oder mehrerer Eigenschaften des Patienten.

10. System nach einem der Ansprüche 1 bis 9, wobei zum Bestimmen der kinematischen Zielbewegungsachse, die sich von der aktuellen kinematischen Bewegungsachse unterscheidet, die Verarbeitungsschaltung konfiguriert ist zum Klassifizieren des Knöchels des Patienten als hypermobil oder hypomobil.

11. System nach einem der Ansprüche 1 bis 10, wobei zum Bestimmen der kinematischen Zielbewegungsachse, die sich von der aktuellen kinematischen Bewegungsachse unterscheidet, die Verarbeitungsschaltung konfiguriert ist zum Identifizieren, dass der Knöchel des Patienten auf der Grundlage der aktuellen kinematischen Bewegungsachse mindestens eines von einer unzureichenden Plantarflexion, einer übermäßigen koronalen Neigung oder einer übertriebenen Kompensation des benachbarten Gelenks aufweist.

12. System nach einem der Ansprüche 1 bis 11, wobei der empfohlene chirurgische Eingriff einen Operationsplan umfasst, der mindestens eines von einer Größe, einer Form und einer Platzierung eines Implantats festlegt.

13. System nach einem der Ansprüche 1 bis 12, wobei der empfohlene chirurgische Eingriff einen Operationsplan umfasst, um mindestens einen Bereich des Weichgewebes des Patienten zu modifizieren, und/oder
wobei der empfohlene chirurgische Eingriff einen Operationsplan zum Korrigieren mindestens eines Knochens umfasst, der einer Knochenverletzung zugeordnet ist.

14. System nach einem der Ansprüche 1 bis 13, wobei die Verarbeitungsschaltlogik ferner konfiguriert ist zum:
während der empfohlene chirurgische Eingriff durchgeführt wird, Bewerten einer kinematischen Zwischenbewegungsachse, die dem Knöchel zugeordnet ist, zum Bestimmen, ob die kinematische Zwischenbewegungsachse mit der kinematischen Zielbewegungsachse ausgerichtet ist;
Identifizieren einer oder mehrerer Ursachen für Bewegungseinschränkungen, die verhindern, dass die kinematische Zwischenbewegungsachse mit der kinematischen Zielbewegungsachse übereinstimmt, auf der Grundlage der kinematischen Zwischenbewegungsachse; und
Ändern des empfohlenen chirurgischen Eingriffs auf der Grundlage einer Differenz zwischen der kinematischen Zwischenbewegungsachse und der kinematischen Zielbewegungsachse.

15. System nach einem der Ansprüche 1 bis 14, ferner umfassend eine Visualisierungsvorrichtung zum Darstellen, während einer Operation, über ein Mixed-Reality-Headset, einer grafischen Darstellung des empfohlenen chirurgischen Eingriffs.

## Revendications

1. Système comprenant :
un circuit de traitement (102) configuré pour :
recevoir des données spécifiques à un patient (108) indicatives d'un mouvement préopératoire associé à une cheville (200) d'un patient ;
déterminer, sur la base des données spécifiques au patient, un axe cinématique actuel (302) du mouvement associé à la cheville ;
déterminer un axe cinématique cible (304) de mouvement pour la cheville, l'axe cinématique cible étant différent de l'axe cinématique actuel ;
générer une fonction de transformée entre l'axe cinématique actuel et l'axe cinématique cible ; et
générer, sur la base de la fonction de transformée, une intervention chirurgicale recommandée qui ajuste le tissu associé à la cheville pour que l'axe cinématique cible de mouvement soit obtenu.

2. Système selon la revendication 1, dans lequel le circuit de traitement est en outre configuré pour :
analyser le mouvement préopératoire associé à la cheville du patient d'après des données de mouvement obtenues à partir de données de capteurs inertiels et de vidéo, capturées alors que le patient se déplace ; et
générer, sur la base de l'analyse du mouvement préopératoire associé à la cheville, les données spécifiques au patient.

3. Système selon l'une quelconque des revendications 1 et 2, dans lequel le circuit de traitement est en outre configuré pour générer les données spécifiques au patient à partir d'une pluralité d'images de la cheville du patient dans différentes positions respectives.

4. Système selon la revendication 3, dans lequel chaque image parmi la pluralité d'images de la cheville est capturée alors que la cheville est soumise à une charge par le patient.

5. Système selon la revendication 3, dans lequel les différentes positions respectives comprennent :
une première position dans laquelle le pied associé à la cheville est en position de dorsiflexion,
une deuxième position dans laquelle le pied est en position neutre, et
une troisième position dans laquelle le pied est en position de flexion plantaire.

6. Système selon l'une quelconque des revendications 3 à 5, dans lequel, pour déterminer l'axe cinématique actuel du mouvement associé à la cheville, le circuit de traitement est configuré pour :
calculer la transformée relative du tibia et de l'astragale dans chaque position parmi les différentes positions respectives ; et
calculer, sur la base de la transformée relative, l'axe cinématique actuel du mouvement associé à la cheville.

7. Système selon l'une quelconque des revendications 1 à 6, dans lequel, pour générer les données spécifiques au patient, le circuit de traitement est configuré pour modifier un modèle tridimensionnel normalisé de pied et de cheville pendant un cycle de marche en un modèle spécifique au patient sur la base d'une pluralité d'images de la cheville du patient.

8. Système selon la revendication 7, dans lequel le modèle spécifique au patient incorpore les conditions de charge externes et les forces musculaires subies par la cheville du patient pendant le cycle de marche.

9. Système selon l'une quelconque des revendications 1 à 8, dans lequel, pour déterminer l'axe cinématique cible de mouvement qui est différent de l'axe cinématique actuel de mouvement, le circuit de traitement est configuré pour sélectionner, sur la base d'une ou plusieurs caractéristiques du patient, l'axe cinématique cible de mouvement à partir d'une base de données d'axes cinématiques potentiels de mouvement.

10. Système selon l'une quelconque des revendications 1 à 9, dans lequel, pour déterminer l'axe cinématique cible de mouvement qui est différent de l'axe cinématique actuel de mouvement, le circuit de traitement est configuré pour classer la cheville du patient comme soit hypermobile soit hypomobile.

11. Système selon l'une quelconque des revendications 1 à 10, dans lequel, pour déterminer l'axe cinématique cible de mouvement qui est différent de l'axe cinématique actuel de mouvement, le circuit de traitement est configuré pour identifier que la cheville du patient présente, sur la base de l'axe cinématique actuel de mouvement, au moins l'une parmi une flexion plantaire insuffisante, une inclinaison coronale excessive, et une compensation exagérée d'une articulation adjacente.

12. Système selon l'une quelconque des revendications 1 à 11, dans lequel l'intervention chirurgicale recommandée comprend un plan chirurgical qui spécifie au moins l'un parmi la taille, la forme, et l'emplacement d'un implant.

13. Système selon l'une quelconque des revendications 1 à 12, dans lequel l'intervention chirurgicale recommandée comprend un plan chirurgical pour modifier au moins une région de tissu mou du patient, et/ou
dans lequel l'intervention chirurgicale comprend un plan chirurgical pour corriger au moins un os associé à un conflit osseux.

14. Système selon l'une quelconque des revendications 1 à 13, dans lequel le circuit de traitement est en outre configuré pour :
alors que l'intervention chirurgicale recommandée est en cours de réalisation, évaluer un axe cinématique intermédiaire de mouvement associé à la cheville pour déterminer si l'axe cinématique intermédiaire de mouvement s'aligne avec l'axe cinématique cible de mouvement ;
identifier, sur la base de l'axe cinématique intermédiaire de mouvement, une ou plusieurs sources de limitations de mouvement qui empêchent l'axe cinématique intermédiaire de mouvement de correspondre à l'axe cinématique cible de mouvement ; et
modifier l'intervention chirurgicale recommandée sur la base de la différence entre l'axe cinématique intermédiaire de mouvement et l'axe cinématique cible de mouvement.

15. Système selon l'une quelconque des revendications 1 à 14, comprenant en outre un dispositif de visualisation pour présenter, pendant une opération, via un casque de réalité mixte, une représentation graphique de l'intervention chirurgicale recommandée.
